# EUROPEAN PATENT APPLICATION

(11) **EP 4 410 949 A1**
(43) Date of publication of application: **07.08.2024**
(21) Application number: 22875049.3
(22) Date of filing: 29.09.2022
(51) Int. Cl.: C12M 1/34, C12Q 1/6869, G01N 27/327, G01N 27/48

(54) **ULTRA-SMALL AREA MICRO-CURRENT DETECTION CIRCUIT UNIT AND SYSTEM**

(30) Priority: 30.09.2021 CN 202111160649
(71) Applicant: Geneus Technologies (Chengdu) Co., Ltd., Chengdu, Sichuan 610041 (CN)
(72) Inventor: ZHANG, Fengti, Chengdu, Sichuan 610041 (CN); JIANG, Ke, Chengdu, Sichuan 610041 (CN); SU, Yunpeng, Chengdu, Sichuan 610041 (CN); ZOU, Yaozhong, Chengdu, Sichuan 610041 (CN)
(74) Representative: Wenzel Nemetzade Warthmüller Patentanwälte Part mbB
(86) International application number: PCT/CN2022/122449
(87) International publication number: WO 2023/051666

(57) **Abstract**

A nanopore sequencing circuit unit (200) and a gene sequencing device. The nanopore sequencing circuit unit (200) is implemented by using a CMOS circuit, and is used for detecting a bidirectional micro-current signal of a nanopore (201). The circuit unit comprises: a nanopore clamping circuit, which is used for stabilizing the voltage of a detection electrode positioned on one side of the nanopore (201), so as to generate a fixed voltage difference with a common electrode (202) positioned on the other side of the nanopore (201), and to form a charging and discharging path with an integral reset circuit, driving individual nucleotide molecules to pass through the nanopore (201) one by one by means of the voltage difference; the integral reset circuit, which is used for, when the nanopore clamping circuit constitutes the charging and discharging path, performing integral amplification on a bidirectional micro-current signal of the nanopore (201) , and converting the bidirectional micro-current signal into a voltage signal; and an output circuit, which is used for receiving the voltage signal converted by the integral reset circuit, and outputting the voltage signal. A high-throughput gene sequencing device having bidirectional detection capability is constructed on the basis of a small-area sequencing circuit unit, such that the detection precision and efficiency are improved.

## Description

### Field of the Invention

The present disclosure relates to a field of electronic circuit technology, and particularly to a nanopore sequencing circuit unit and a gene sequencing device, which can be used for the detection of biological micro-current signals for gene sequencing and pA-level micro-currents in other application fields.

### Background of the Invention

Nanopore sequencing adopts electrophoresis technology by driving individual molecules through a nanopore one by one utilizing electrophoresis to realize sequencing. Currently, nanopore sequencing technology has gradually been widely used in research on DNA sequencing, disease detection, drug screening, environmental monitoring, or the like. In the existing technology, sequencing is performed merely in one direction, and charges are supplemented to the electrode in the other direction only. Sequencing efficiency and precision can be greatly improved if both directions are properly utilized. In addition, throughput of a currently known Roche gene sequencing device reaches the order of millions. Integrating so many basic sequencing units in one system places extremely strict requirements on the area of the sequencing circuit unit. And competitiveness of a product can be significantly improved by a small-area basic sequencing unit. Whether this technology can be developed into chip-based and high-throughput quantification limits the application of the nanopore sequencing technology, and therefore, a sequencing circuit unit with a smaller area is desired to solve this problem.

### Summary of the invention

In view of this, a nanopore sequencing circuit unit and a gene sequencing device are provided in the present disclosure, which are used to construct a high-throughput gene sequencing device having bidirectional detection capability on the basis of a small-area sequencing circuit unit, such that detection precision and efficiency can be improved.

In a first aspect, a nanopore sequencing circuit unit is provided in the present disclosure, which is implemented by using a CMOS circuit and configured to detect a bidirectional micro-current signal of the nanopore. The nanopore sequencing circuit unit comprises:
a nanopore clamping circuit for stabilizing a voltage of a detection electrode located on one side of the nanopore to generate a fixed voltage difference between both ends of the nanopore with a common electrode located on the other side of the nanopore, forming charging and discharging paths with an integral reset circuit, and driving individual nucleotide molecules through the nanopore one by one by using the voltage difference;
the integral reset circuit for integrally amplifying the bidirectional micro-current signal of the nanopore and converting it into a voltage signal, when forming the charging and discharging paths with the nanopore clamping circuit; and
an output circuit for receiving the voltage signal converted by the integral reset circuit, and outputting the voltage signal.

In an alternative embodiment, the nanopore clamping circuit includes an operational amplifier circuit, a first clamp transistor and a second clamp transistor; wherein the operational amplifier circuit outputs a first stage of voltage to make the first clamp transistor conductive to form the charging path, and outputs a second stage of voltage to make the second clamp transistor conductive to form the discharging path.

In an alternative embodiment, the operational amplifier circuit includes a first stage circuit and a second stage circuit, the first clamp transistor includes a first transistor and a second transistor connected in series, the second clamp transistor includes a third transistor and a fourth transistor connected in series, the first stage circuit outputs the first stage of voltage to an input terminal of the second stage circuit and a control terminal of the first transistor, and the second stage circuit outputs the second stage of voltage to a control terminal of the third transistor.

In an alternative embodiment, the operational amplifier circuit further includes a positive input terminal and a negative input terminal, the positive input terminal is connected to a common level, and the negative input terminal is connected to the detection electrode.

In an alternative embodiment, the operational amplifier circuit further includes a bias voltage input terminal for inputting a bias voltage to the first stage circuit and the second stage circuit.

In an alternative embodiment, the first stage circuit includes a five-transistor operational amplifier and the second stage circuit includes a first source follower.

In an alternative embodiment, a drain of the first transistor is connected to a source of the second transistor, and a drain of the third transistor is connected to a source of the fourth transistor; a source of the first transistor is connected to a source of the third transistor and connected to the detection electrode; a drain of the second transistor is connected to a drain of the fourth transistor and connected to the integral reset circuit; a control terminal of the second transistor is connected to a control terminal of the fourth transistor and connected to a switching control signal.

In an alternative embodiment, the switching control signal controls the nanopore clamping circuit to switch between a charging state and a discharging state.

In an alternative embodiment, the first transistor and the second transistor include PMOS transistors, the third transistor and the fourth transistor include NMOS transistors, and the second stage of voltage output to the control terminal of the third transistor is greater than the first stage of voltage output to the control terminal of the first transistor.

In an alternative embodiment, the integral reset circuit includes an integrating capacitor and a first reset switch, a first terminal of the integrating capacitor is connected to a first terminal of the first reset switch, and also connected to the drain of the second transistor and the drain of the fourth transistor, and a second terminal of the integrating capacitor is grounded; a second terminal of the first reset switch is connected to a pre-reset voltage for periodically resetting the voltage of the integrating capacitor.

In an alternative embodiment, the output circuit includes a second source follower and a selection switch, the second source follower has an input terminal connected to the first terminal of the integrating capacitor, and an output terminal connected to a first terminal of the selection switch, and a second terminal of the selection switch outputs the voltage signal converted by the integral reset circuit to a common signal line.

In an alternative embodiment, the nanopore sequencing circuit unit further includes a second reset switch, the second reset switch has a first terminal connected to the detection electrode, and a second terminal connected to the common level, so as to fix the voltage of the detection electrode at the common level when the nanopore clamping circuit switches between the charging state and the discharging state.

In a second aspect, a gene sequencing device is further provoded in the present disclosure, including a chip integrating a plurality of microporous structure units and a plurality of nanopore sequencing circuit units according to any of the aforementioned embodiments, the microporous structure units include nanopores, and common electrodes and detection electrodes located on both sides of the nanopores; the plurality of nanopore sequencing circuit units are connected to the plurality of microporous structure units correspondingly for measuring the bidirectional micro-current signals of the nanopores in the corresponding microporous structure units.

In an alternative embodiment, a common signal line and an analog-to-digital conversion circuit connected to the common signal line are further included, the common signal line is used to receive a voltage signal output by the nanopore sequencing circuit unit, and the analog-to-digital conversion circuit is used to convert the voltage signal into a digital signal.

In an alternative embodiment, a common tail current source is further included, one terminal of the common tail current source is connected to the common signal line and the other terminal is grounded.

In an alternative embodiment, the chip includes a MEMS chip implementing the microporous structural unit.

The nanopore sequencing circuit unit according to the present disclosure has an extremely small circuit area, and taking a 180 nm-CMOS process as an example, the area can be compressed to be within 100 µm², which is suitable for the integral construction of a nanopore gene sequencing device with a throughput of millions or even tens of millions, and can greatly improve the degree of integration, thereby achieving high throughput and high detection efficiency. In addition, the nanopore sequencing circuit unit and the gene sequencing device with the same have the bidirectional detection capability, which can further correct errors, thereby reducing the error rate and improving the detection precision.

### Brief Description of the Drawings

To illustrate the technical solutions in the examples of the present disclosure or the existing technology more clearly, the following briefly describes the accompanying drawings required for describing the embodiments or the existing technology. Apparently, the accompanying drawings illustrated below show some of the examples of the present disclosure, and a person of ordinary skill in the art may still obtain further drawings from these accompanying drawings without inventive efforts.
FIG. 1 is a schematic diagram of a nanopore equivalent model 100 adopted in an embodiment of the present disclosure;
FIG. 2 is a schematic circuit diagram of a nanopore sequencing circuit unit 200 according to Example 1 of the present disclosure;
FIG. 3 is a schematic circuit diagram of a nanopore sequencing circuit unit 300 according to Example 2 of the present disclosure;
FIG. 4 is a schematic circuit diagram of a micro-current detection circuit according to Example 1 of the present disclosure;
FIG. 4A is a schematic diagram of a circuit working state of the nanopore sequencing circuit unit according to an example of the present disclosure during discharging;
FIG. 4B is a schematic diagram of a circuit working state of the nanopore sequencing circuit unit according to an example of the present disclosure during charging;
FIG. 5A is a schematic diagram of a working waveform of the nanopore sequencing circuit unit according to Example 1 of the present disclosure;
FIG. 5B is a schematic diagram of a working waveform of the nanopore sequencing circuit unit according to Example 2 of the present disclosure; and
FIG. 6 is a schematic structural diagram of a gene sequencing device according to an example of the present disclosure.

### Detailed Description

To make the objectives, technical solutions and advantages of the examples of the present disclosure clearer, the technical solutions of the examples of the present disclosure will be described below clearly and completely with reference to the accompanying drawings in the examples of the present disclosure. Apparently, the described examples are some but not all of the examples of the present disclosure. All other examples obtained by a person of ordinary skill in the art based on the examples of the present disclosure without inventive efforts shall fall within the protection scope of the present disclosure.

In the present disclosure, it is to be understood that the terms, such as "comprise/comprising", "include/including", "have/having", or the like, are intended to indicate the presence of the disclosed features, numerals, steps, behaviors, components, parts, or combinations thereof in the specification, and are not intended to exclude the possibility of the presence or addition of one or more other features, numerals, steps, behaviors, components, parts, or combinations thereof.

In a nanopore sequencing device, nucleotide molecules are usually driven through a nanopore by means of a voltage applied to two ends of a test chamber, and the type of the nucleotide molecules passing through the nanopore is detected by detecting a micro-current characteristic signal output by the nanopore, so as to realize gene sequencing.

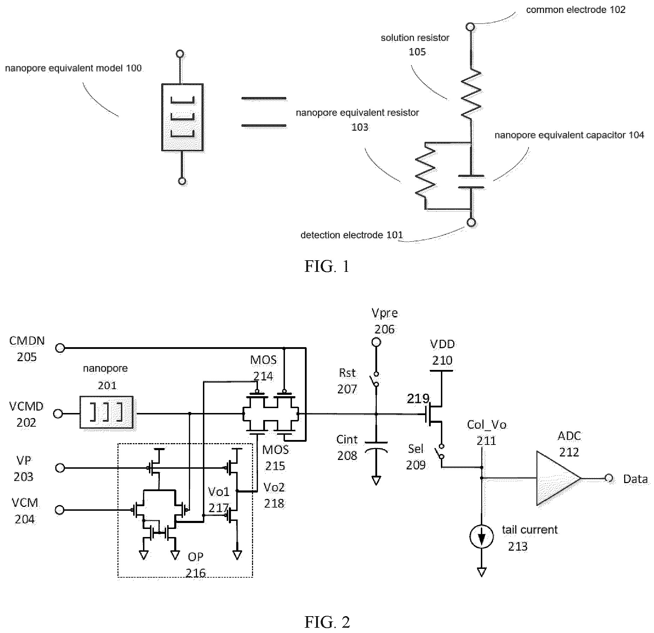

FIG. 1 is a schematic diagram of a nanopore equivalent model 100 adopted in an example of the present disclosure. As shown in FIG. 1, electrical characteristics of a nanopore can be simulated by using a nanopore equivalent resistor 103 and a nanopore equivalent capacitor 104, which is equivalent to the electrical characteristics obtained by connecting the parallel structure of the nanopore equivalent resistor 103 and the nanopore equivalent capacitor 104 in series with a solution resistor 105.

FIG. 2 is a schematic circuit diagram of a nanopore sequencing circuit unit 200 according to Example 1 of the present disclosure. As shown in FIG. 2, the nanopore sequencing circuit unit 200 is implemented by a CMOS circuit, including a nanopore clamping circuit, an integral reset circuit and an output circuit.

In this example, the nanopore clamping circuit is configured to stabilize a voltage of a detection electrode of a nanopore 201 to generate a fixed voltage difference between both ends of the nanopore 201 with a common electrode VCMD 202 located on the other side of the nanopore 201, forming charging and discharging paths with the integral reset circuit, and driving individual nucleotide molecules through the nanopore one by one by using the voltage difference between both ends of the nanopore 201. The integral reset circuit is configured to integrally amplifying the bidirectional micro-current signal of the nanopore 201 and converting it into a voltage signal when forming the charging and discharging paths with the nanopore clamping circuit. The output circuit is configured to receive the voltage signal converted by the integral reset circuit, and to output the voltage signal.

In some embodiments, the nanopore clamping circuit includes an operational amplifier circuit 216, a first clamp transistor 214 and a second clamp transistor 215.

In those embodiments, the operational amplifier circuit 216 includes a first stage circuit and a second stage circuit. Exemplarily, the first stage circuit may include a five-transistor (5T) operational amplifier; the second stage circuit may include a source follower, and a source follower with two transistors connected in series is exemplarily shown in the drawing. The first clamp transistor 214 includes a first transistor and a second transistor connected in series, and the second clamp transistor 215 includes a third transistor and a fourth transistor connected in series.

The operational amplifier circuit 216 has 5 terminals which are a bias voltage input terminal VP 203, a positive input terminal, a negative input terminal, a first stage circuit output terminal, and a second stage circuit output terminal respectively, wherein the positive input terminal is connected to a common level VCM 204, and the negative input terminal is connected to the detection electrode of the nanopore 201. The bias voltage input terminal VP 203 inputs a bias voltage to both the first stage circuit and the second stage circuit. The first stage circuit output terminal outputs a voltage Vo1 217, which is connected to an input terminal of the second stage circuit and is also connected to a control terminal of the first transistor of the first clamp transistor 214, and the second stage circuit output terminal outputs a voltage Vo2 218, which is connected to a control terminal of the third transistor of the second clamp transistor 215.

In some embodiments, the first transistor and the second transistor included in the first clamp transistor 214 may be PMOS transistors. The first clamp transistor 214 includes 4 terminals which are a source of the first transistor, the control terminal of the first transistor, a control terminal of the second transistor, and a drain of the second transistor respectively, and a drain of the first transistor is connected to a source of the second transistor. The third transistor and the fourth transistor included in the second clamp transistor 215 may be NMOS transistors. The second clamp transistor 215 also includes 4 terminals which are a source of the third transistor, the control terminal of the third transistor, a control terminal of the fourth transistor, and a drain of the fourth transistor respectively, and a drain of the third transistor is connected to a source of the fourth transistor. The source of the first transistor of the first clamp transistor 214 and the source of the third transistor of the second clamp transistor 215 are connected with each other and connected to the detection electrode of the nanopore 201; the drain of the second transistor of the first clamp transistor 214 and the drain of the fourth transistor of the second clamp transistor 215 are connected with each other and connected to the integral reset circuit. The control terminal of the second transistor of the first clamp transistor 214 and the control terminal of the fourth transistor of the second clamp transistor 215 are connected with each other and connected to a switching control signal CMDN 205.

In some embodiments, the output terminal of the first stage circuit and the output terminal of the second stage circuit of the operational amplifier circuit 216 control the first clamp transistor 214 and the second clamp transistor 215 to form the charging path and the discharging path respectively. The output terminal of the first stage circuit outputs the voltage Vo1 217 to make the first clamp transistor 214 conductive to form the charging path, and the output terminal of the second stage circuit outputs the voltage Vo2 218 to make the second clamp transistor 215 conductive to form the discharging path.

In some embodiments, the integral reset circuit may include an integrating capacitor 208 and a reset switch 207. A first terminal (i.e., a charging terminal) of the integrating capacitor 208 is connected to a first terminal of the reset switch 207, the other terminal of the integrating capacitor 208 is grounded, and a second terminal of the reset switch 207 is connected to a pre-reset voltage Vpre 206. The charging terminal of the integrating capacitor 208 is also connected to the drain of the second transistor of the first clamp transistor 214 and the drain of the fourth transistor of the second clamp transistor 215, for integrally amplifying the micro-current signal of the nanopore to be detected and converting it into the voltage signal. The reset switch 207 is configured to periodically clear and reset a voltage of the integrating capacitor 208 by means of a reset signal Rst.

In some embodiments, when the switching control signal CMDN 205 is at a high level, the discharging path is turned on (conductive) and the charging path is turned off; when the switching control signal CMDN 205 is at a low level, the charging path is turned on (conductive) and the discharging path is turned off. The charging and the discharging paths transfer a current of the nanopore to the integrating capacitor 208, and the current is output through the output circuit after a fixed time integral amplification.

In some embodiments, the output circuit includes a source follower 219 and a selection switch 209. An input terminal of the source follower 219 is connected to the first terminal (i.e., the charging terminal) of the integrating capacitor 208 to receive the voltage signal output from the integral reset circuit, and is connected to the common signal line 211 through the selection switch 209.

In some embodiments, the common signal line 211 is connected to a tail current source 213 in parallel and connected to an analog-to-digital converter 212. The analog-to-digital converter 212 is configured to receive an output voltage signal of the source follower 219 and convert it into a digital code value, and it has a multiplexing function and can be multiplexed among a plurality of nanopore sequencing circuit units.

The nanopore sequencing circuit unit according to an example of the present disclosure is implemented by adopting the CMOS circuit, has an extremely small circuit area, facilitates high-throughput integration, has charging-discharging bidirectional detection capability, and can significantly improve sequencing precision and efficiency when used in a high-throughput gene sequencing device.

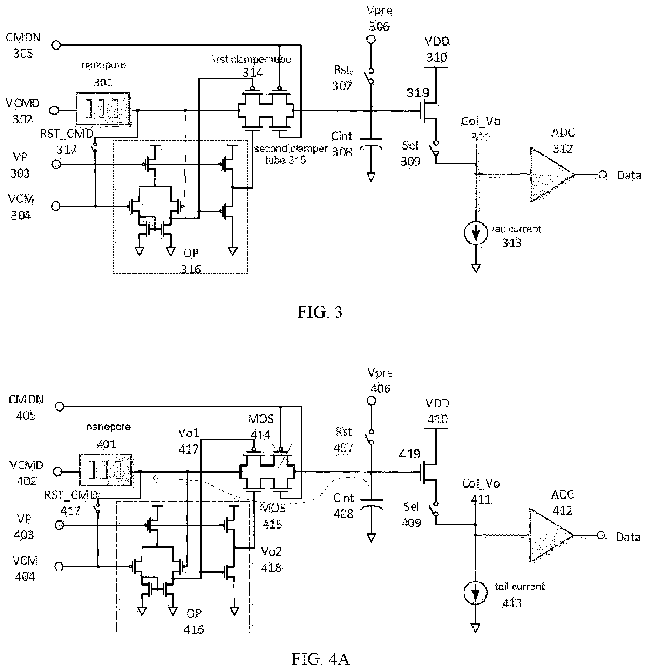

FIG. 3 is a schematic circuit diagram of a nanopore sequencing circuit unit 300 according to Example 2 of the present disclosure. As shown in FIG. 3, a reset switch 317 is connected in series between the detection electrode of the nanopore 301 and the common level VCM 304 in the nanopore sequencing circuit unit 300 of the present example, based on the circuit structure of Example 1. The addition of the reset switch 317 can fix the detection electrode of the nanopore at the common level when switching the charging and discharging direction of the circuit, which is conducive to the quick establishment of a working point of the circuit, and improving the response speed of the circuit during bidirectional detection and switching.

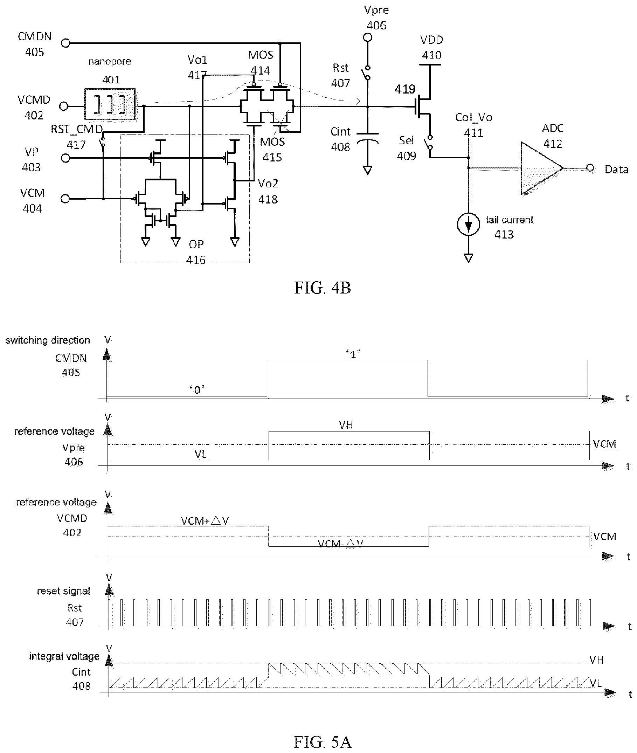

FIGS. 4A and 4B are schematic diagrams of circuit working states of the nanopore sequencing circuit unit according to an example of the present disclosure during discharging and charging.

As shown in FIG. 4A, when the switching control signal CMDN 405 is at a high level and the circuit works in a discharging state, the first clamp transistor MOS 414 is in an open circuit state, the second clamp transistor MOS 415 is turned on (conductive), the operational amplifier circuit 416, the second clamp transistor MOS 415 and the nanopore 401 form a negative feedback loop to stabilize the end voltage of the nanopore, and at this point, the current flows to the nanopore from the integrating capacitor 408, is converted into the voltage signal after integral amplification, and is output through the source follower 419 for sampling analog-to-digital conversion.

As shown in FIG. 4B, when the switching control signal CMDN 405 is at a low level and the circuit works in a charging state, the second clamp transistor MOS 415 is in an open circuit state, the first clamp transistor MOS 414 is turned on (conductive), the operational amplifier circuit 416, the first clamp transistor MOS 414 and the nanopore 401 form a negative feedback loop to stabilize the end voltage of the nanopore, and at this point, the current flows from the nanopore 401 to the integrating capacitor 408, is converted into the voltage signal after integral amplification, and is output through the source follower 419 for sampling analog-to-digital conversion.

As shown in FIGS. 4A and 4B, an output voltage Vo1 417 of the first stage circuit of the operational amplifier 416 is connected to the control terminal of the first transistor of the first clamp transistor 414, an output voltage Vo2 418 of the second stage circuit is connected to the control terminal of the third transistor of the second clamp transistor 415, and Vo2 has the same polarity but a higher voltage compared with Vo1. When the circuit works in the charging state, the operational amplifier 416 is required to make the first clamp transistor MOS 414 turn on (conductive), and when the first clamp transistor MOS 414 is a PMOS transistor, it needs a lower voltage to turn on, and can be controlled by Vo1; when the circuit works in the discharging state, the operational amplifier 416 is required to make the second clamp transistor MOS 415 to turn on (conductive), and when the second clamp transistor MOS 415 is an NMO S transistor, the operational amplifier 416 is required to output a higher voltage to turn on the second clamp transistor MOS 415, and at this point, Vo2 is used.

FIG. 5A is a schematic diagram of a working waveform of the nanopore sequencing circuit unit according to Example 1 of the present disclosure. As shown in FIG. 5A, when the switching control signal CMDN 405 is '0', the circuit works in the charging state; when the switching control signal CMDN 405 is '1', the circuit works in the discharging state.

The pre-reset voltage Vpre 406 is a voltage VL during charging and a voltage VH during discharging, and the voltages VH and VL are at upper and lower sides of the common level VCM respectively. A reference voltage VCMD 402 of the common electrode of the nanopore is centered on the common level VCM and varies up and down by a fixed voltage difference ΔV Since the reset signal Rst of the reset switch periodically resets the integrating capacitor 408 in FIG. 4 with a reset voltage value being the pre-reset voltage Vpre, the voltage of the integrating capacitor 408 rises from the voltage VL during charging and falls from the voltage VH during discharging with the pre-reset voltage Vpre as the boundary. It should be noted that the voltages shown in FIG. 5A can be flexibly adjusted according to system requirements.

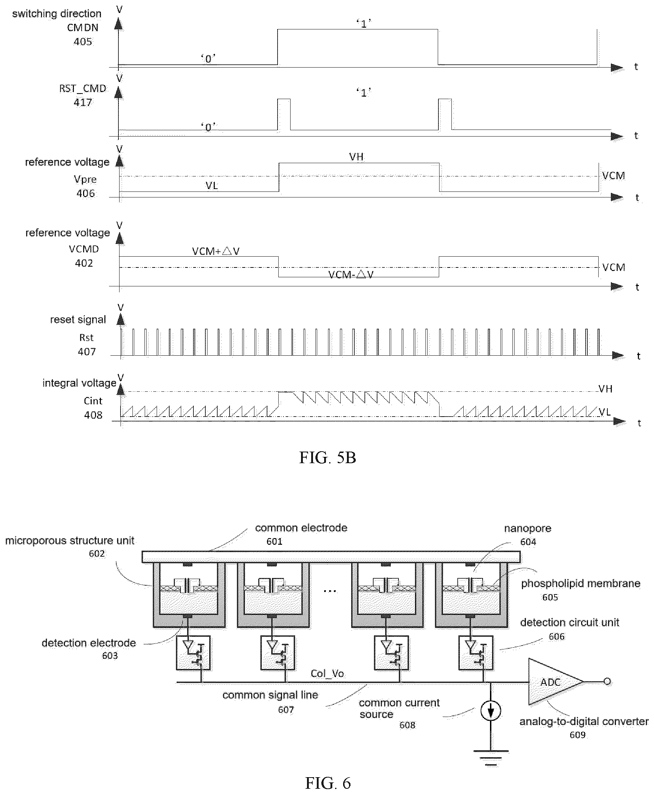

FIG. 5B is a schematic diagram of a working waveform of the nanopore sequencing circuit unit according to Example 2 of the present disclosure. As shown in FIG. 5B, if a circuitry system is required to be stabilized quickly, a RST_CMD signal of the reset switch 417 can be enabled (see FIGS. 4A and 4B), the RST_CMD signal is turned on at the transition point of the switching control signal CMDN 405 to switch on the reset switch 417, and the detection electrode (clamping terminal) of the nanopore is directly connected to the common level VCM, and the voltage of the detection electrode of the nanopore can be stabilized quickly compared to the manner of the preceding example.

FIG. 6 is a schematic structural diagram of a gene sequencing device according to an example of the present disclosure. As shown in FIG. 6, the gene sequencing device according to the present example includes a plurality of microporous structure units 602 integrated and a plurality of nanopore sequencing circuit units 606 according to any of the preceding examples, and the microporous structure units 602 and the nanopore sequencing circuit units 606 are in one-to-one correspondence. Each microporous structure unit 602 includes a nanopore 604, and a common electrode 601 and a detection electrode 603 located on both sides of the nanopore 604. In the device, the microporous structure units 602 can be implemented using a MEMS technology, and the microporous structure units 602 and the corresponding nanopore sequencing circuit units 606 are integrated on the same chip, thereby forming the high-throughput gene sequencing device.

In some embodiments, the plurality of microporous structure units 602 may share one common electrode 601, and output voltages of the plurality of nanopore sequencing circuit units 606 may be output to a shared common signal line 607 through a selection switch, and converted into digital signals by an analog-to-digital converter 609, and then output, so as to realize multiplexing of an analog-to-digital conversion function of the output voltage signals of the nanopore sequencing circuit units 606.

In some embodiments, the gene sequencing device further includes a common tail current source 608, one terminal of the common tail current source 608 is connected to the common signal line 607 and the other terminal is grounded.

The nanopore sequencing circuit unit according to the example of the present disclosure may be implemented by a CMOS circuit, and has an extremely small circuit area, and taking a 180 nm-CMOS process as an example, the area can be compressed to be within 100µm², which is suitable for the integral construction of a nanopore gene sequencing device with a throughput of millions or even tens of millions, and can be greatly improved the degree of integration, thereby achieving high throughput and the high detection efficiency. In addition, the nanopore sequencing circuit unit according to the embodiment of the present disclosure has bidirectional detection capability, which can further reduce the error rate, and improves sequencing detection precision.

It should be noted that the above embodiments can be freely combined as required, the devices involved in the circuit are illustrated as CMOS devices, while other devices, such as BJTs, JFETs, or the like, can also implement the technical solution of the present disclosure. The above are only preferred embodiments of the present disclosure, and it should be noted that variations and improvements can be made by those skilled in the art without departing from the principles of the present disclosure, and these variations and improvements should be considered as falling within the scope of the present disclosure.

## Claims

1. A nanopore sequencing circuit unit, **characterized in that** the nanopore sequencing circuit unit is implemented by using a CMOS circuit and configured to detect a bidirectional micro-current signal of a nanopore, which comprises:
a nanopore clamping circuit for stabilizing a voltage of a detection electrode located on one side of the nanopore to generate a fixed voltage difference between both ends of the nanopore with a common electrode located on the other side of the nanopore, forming charging and discharging paths with an integral reset circuit, and driving individual nucleotide molecules through the nanopore one by one using the voltage difference;
the integral reset circuit for integrally amplifying the bidirectional micro-current signal of the nanopore and converting it into a voltage signal, when forming the charging and discharging paths with the nanopore clamping circuit; and
an output circuit for receiving the voltage signal converted by the integral reset circuit, and outputting the voltage signal.

2. The nanopore sequencing circuit unit according to claim 1, **characterized in that** the nanopore clamping circuit comprises an operational amplifier circuit, a first clamp transistor and a second clamp transistor; wherein the operational amplifier circuit is configured to output a first stage of voltage to make the first clamp transistor conductive to form the charging path, and to output a second stage of voltage to make the second clamp transistor conductive to form the discharging path.

3. The nanopore sequencing circuit unit according to claim 2, **characterized in that** the operational amplifier circuit comprises a first stage circuit and a second stage circuit, the first clamp transistor comprises a first transistor and a second transistor connected in series, the second clamp transistor comprises a third transistor and a fourth transistor connected in series, the first stage circuit is configured to output the first stage of voltage to an input terminal of the second stage circuit and a control terminal of the first transistor, and the second stage circuit is configured to output the second stage of voltage to a control terminal of the third transistor.

4. The nanopore sequencing circuit unit according to claim 3, **characterized in that** the operational amplifier circuit further comprises a positive input terminal and a negative input terminal, the positive input terminal is connected to a common level, and the negative input terminal is connected to the detection electrode.

5. The nanopore sequencing circuit unit according to claim 4, **characterized in that** the operational amplifier circuit further comprises a bias voltage input terminal for inputting a bias voltage to the first stage circuit and the second stage circuit.

6. The nanopore sequencing circuit unit according to claim 5, **characterized in that** the first stage circuit comprises a five-transistor operational amplifier and the second stage circuit comprises a first source follower.

7. The nanopore sequencing circuit unit according to any one of claims 4 to 6, **characterized in that** a drain of the first transistor is connected to a source of the second transistor, and a drain of the third transistor is connected to a source of the fourth transistor; a source of the first transistor is connected to a source of the third transistor and connected to the detection electrode; a drain of the second transistor is connected to a drain of the fourth transistor and connected to the integral reset circuit; a control terminal of the second transistor is connected to a control terminal of the fourth transistor and connected to a switching control signal.

8. The nanopore sequencing circuit unit according to claim 7, **characterized in that** the switching control signal is configured to control the nanopore clamping circuit to switch between a charging state and a discharging state.

9. The nanopore sequencing circuit unit according to claim 8, **characterized in that** the first transistor and the second transistor comprise PMOS transistors, the third transistor and the fourth transistor comprise NMOS transistors, wherein the second stage of voltage output to the control terminal of the third transistor is greater than the first stage of voltage output to the control terminal of the first transistor.

10. The nanopore sequencing circuit unit according to claim 9, **characterized in that** the integral reset circuit comprises an integrating capacitor and a first reset switch, a first terminal of the integrating capacitor is connected to a first terminal of the first reset switch, and also connected to the drain of the second transistor and the drain of the fourth transistor, and a second terminal of the integrating capacitor is grounded; a second terminal of the first reset switch is connected to a pre-reset voltage for periodically resetting a voltage of the integrating capacitor.

11. The nanopore sequencing circuit unit according to claim 10, **characterized in that** the output circuit comprises a second source follower and a selection switch, the second source follower has an input terminal connected to the first terminal of the integrating capacitor, and an output terminal connected to a first terminal of the selection switch, and a second terminal of the selection switch is configured to output the voltage signal converted by the integral reset circuit to a common signal line.

12. The nanopore sequencing circuit unit according to any one of claims 8 to 11, **characterized in that** the nanopore sequencing circuit unit further comprises a second reset switch, the second reset switch has a first terminal connected to the detection electrode, and a second terminal connected to the common level, and is configured to fix the voltage of the detection electrode at the common level when the nanopore clamping circuit switches between the charging state and the discharging state.

13. A gene sequencing device, **characterized by** comprising a chip integrating a plurality of microporous structure units and a plurality of nanopore sequencing circuit units according to any one of claims 1 to 12, wherein the microporous structure units comprise nanopores, and common electrodes and detection electrodes located on both sides of the nanopores; the plurality of nanopore sequencing circuit units are connected to the plurality of microporous structure units correspondingly for measuring bidirectional micro-current signals of the nanopores in the corresponding microporous structure units.

14. The gene sequencing device according to claim 13, **characterized by** further comprising a common signal line and an analog-to-digital conversion circuit connected to the common signal line, wherein the common signal line is configured to receive a voltage signal output from the nanopore sequencing circuit unit, and the analog-to-digital conversion circuit is configured to convert the voltage signal into a digital signal.

15. The gene sequencing device according to claim 14, **characterized by** further comprising a common tail current source, wherein one terminal of the common tail current source is connected to the common signal line and the other terminal of the common tail current source is grounded.

16. The gene sequencing device according to claim 13, **characterized in that** the chip comprises a MEMS chip implementing the microporous structural unit.
